# EUROPEAN PATENT SPECIFICATION

(11) **EP 1 852 137 B1**
(45) Date of publication and mention of the grant of the patent: **17.11.2010**
(21) Application number: 07007698.9
(22) Date of filing: 16.04.2007
(51) Int. Cl.: A61M 16/00

(54) **Apparatus for administering oxygen or air with added oxygen, for respiratory therapies**
Vorrichtung zur Verabreichung von Sauerstoff oder damit angereicherter Luft für Atemwegstherapien
Appareil pour administrer de l'oxygène ou de l'air enrichi avec de l'oxygène, pour thérapies respiratoires

(30) Priority: 04.05.2006 IT MI20060158 U
(43) Date of publication of application: 07.11.2007
(73) Proprietor: Starmed S.p.A., 41037 Mirandola MO (IT)
(72) Inventor: Luppi, Libero, 41037 Mirandola (IT)
(74) Representative: Alagem Modiano, Lara S.

(56) References cited:
- EP-A- 1 186 315
- GB-A- 805 941
- US-A- 3 717 147
- US-A- 3 769 973
- US-A- 4 036 253
- US-A- 4 495 946
- US-A- 5 819 728
- US-B2- 6 830 048

## Description

The present invention relates to an apparatus for administering oxygen or air with added oxygen, for respiratory therapies.

As is known, in both hospital and nonhospital environments, respirators are currently employed for emergencies which use apparatuses and/or machines which are designed to deliver adequate flows of air mixed with oxygen to be administered to the patient. With currently available solutions, the apparatus is of the type with fixed installation, since it requires a connection between the air and oxygen sources, and the apparatuses for adjusting the flow and the associated oxygen content.

Accordingly, such apparatus is scarcely versatile and must be subjected to frequent sterilization operations, since the same apparatus must be used for all patients.

GB 805941 discloses an apparatus as defined in the preamble of claim 1.

US-A-5 819 728 discloses a gas treatment hood, but does not disclose a Venturi meter connected thereto.

The aim of the invention is to solve the problems described above by providing an apparatus for administering oxygen or air with added oxygen, for respiratory therapies, which allows to simplify considerably the apparatuses that are currently used until an assembly is obtained which can also be carried easily and therefore is adapted for the most disparate types of use.

Within this aim, an object of the invention is to provide an apparatus in which the elements that are used are of the disposable type, consequently simplifying all the operations linked to the steps for sterilizing the apparatus.

Another object of the present invention is to provide an apparatus which thanks to its particular constructive characteristics is capable of giving the greatest assurances of reliability and safety in use.

Another object of the present invention is to provide an apparatus which can be obtained easily starting from commonly commercially available elements and materials and is also competitive from a merely economical standpoint.

This aim and these and other objects, which will become better apparent hereinafter, are achieved by an apparatus for administering oxygen or air with added oxygen, for respiratory therapies, according to the invention, as defined in claim 1.

Further characteristics and advantages of the present invention will become better apparent from the description of a preferred but not exclusive embodiment of an apparatus for administering oxygen or air with added oxygen, for respiratory therapies, illustrated by way of non-limiting example in the accompanying drawings, wherein:

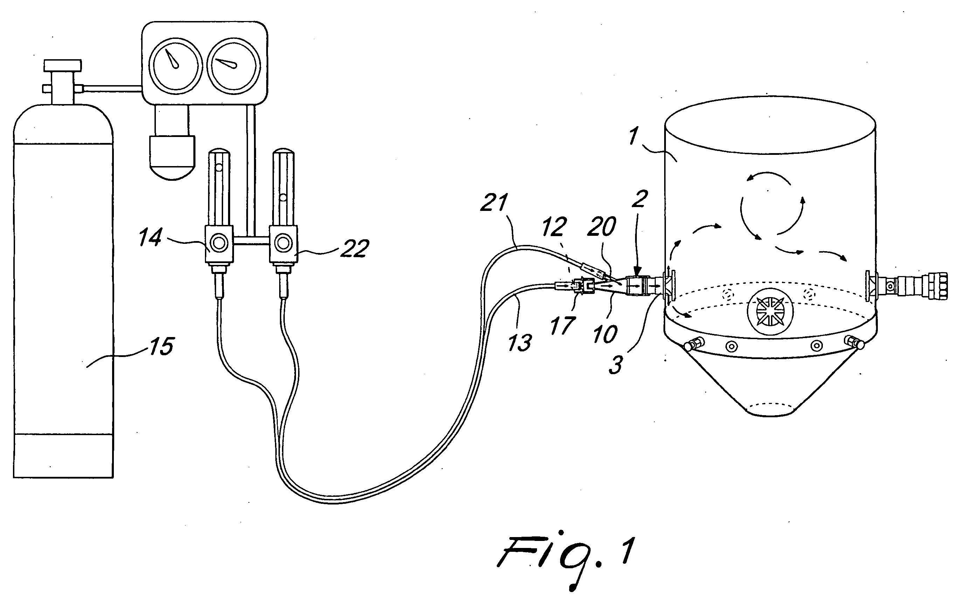

Figure 1 is a schematic view of the apparatus according to the invention;

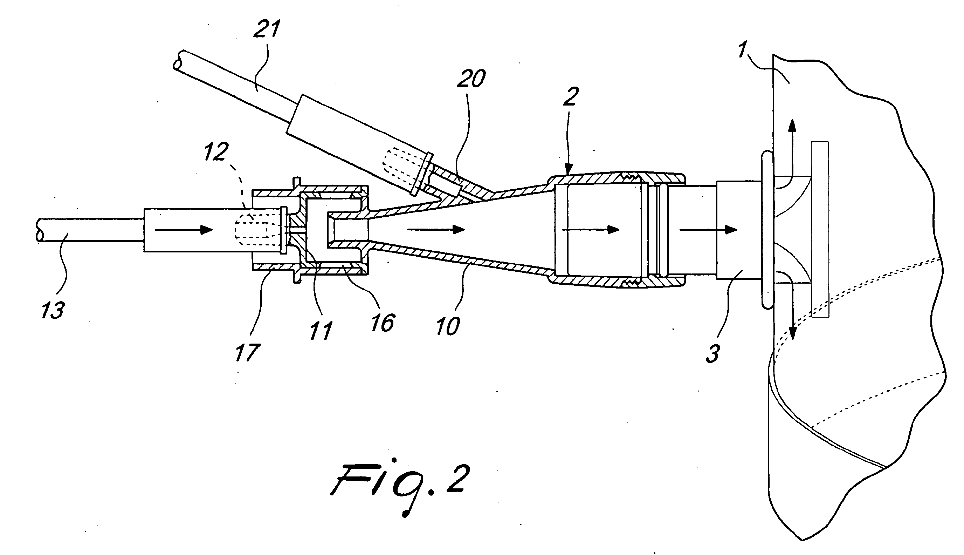

Figure 2 is a sectional view of the Venturi meter in the position for administering only oxygen;

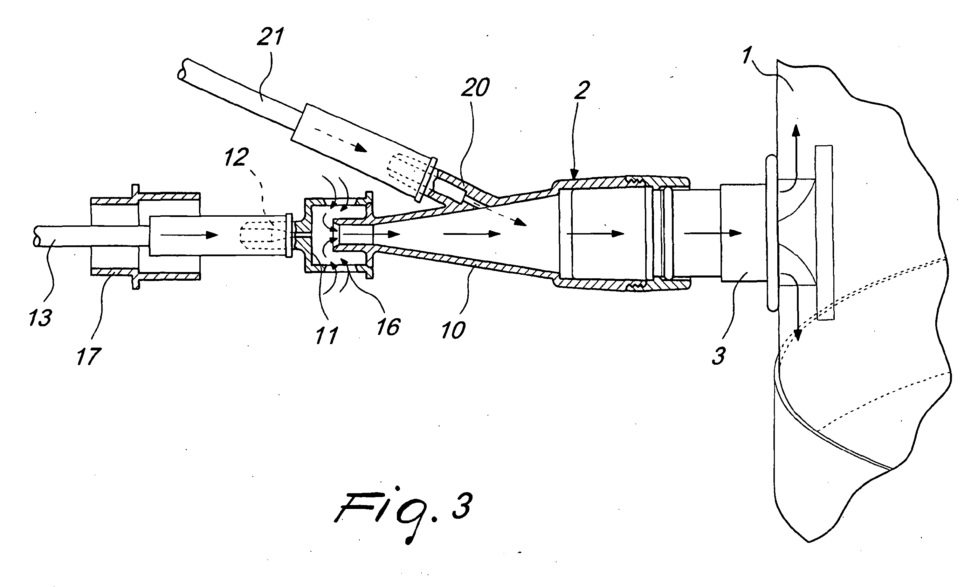

Figure 3 is a view of the Venturi meter in the position for the inflow of oxygen with added air.

With reference to the figures, the apparatus for administering oxygen or air with added oxygen, for respiratory therapies, according to the invention comprises, associated with a respirator hood 1, a Venturi meter, which is generally designated by the reference numeral 2 and further has the important peculiarity of being of the disposable type.

Another important aspect consists in that the Venturi meter 2 is connected directly to the delivery port 3 of the hood 1, which is provided with the usual antisuffocation valve.

The Venturi meter 2 is provided with a body 10, which has, at its intake end, a coupling 11 to a port or intake 12 for connection to a first oxygen delivery hose 13, associated with a first flow meter 14 which is connected, in a per se known manner, to an oxygen source such as a cylinder 15 for dispensing oxygen or to another suitable source.

At the coupling body 11 openings 16 are provided, which are controlled by a flow control element constituted by a cap 17 which can be positioned on the coupling body to block up said openings when one does not wish to add air or can instead be removed to provide the Venturi effect which causes mixing of a preset quantity of air with the oxygen stream.

On the body 10 of the Venturi meter 2 there is also an auxiliary intake port 20 for the connection of a second oxygen delivery duct 21, controlled by a second flowmeter 22, which is also connected to the cylinder 15 or other suitable source.

With the described arrangement, it is therefore possible to adjust at will the mixing between air and oxygen in the preset quantities.

When it is not necessary to mix air and oxygen, it is in fact sufficient for the cap 17 to remain arranged so as to close the openings 16, so that 100% oxygen is supplied to the patient.

By removing the cap 17, a mixing between air and oxygen occurs which is constant in terms of percentage independently of the flow of oxygen, since such percentage is determined by the structure of the Venturi meter, which is designed preferably but not necessarily with a 1:4 ratio.

In order to modify the percentages of oxygen, it is optionally possible to introduce, by means of the second duct 21, additional quantities of oxygen which, by mixing with the oxygen mixed with air that enters through the main intake of the Venturi meter, allow to modify the percentages between air and oxygen.

From what has been described above it is therefore evident that the invention achieves the proposed aim and objects and in particular the fact is stressed that an apparatus is provided in which it is possible to use a Venturi meter of the disposable type which has the characteristic of being connected directly to the hood, so that in addition to being of the portable type the entire structure currently used is simplified, since by integrating in practice the Venturi meter with the hood it is sufficient to have available a cylinder of oxygen or other suitable source to achieve the percentages of mixing between air and oxygen that are required.

Another important aspect further consists in that the Venturi meter, by being made of plastics, can be of the disposable type, thus solving all problems linked to maintenance, calibration and sterilization of the apparatus.

In practice, the materials used, so long as they are compatible with the specific use, as well as the contingent shapes and dimensions, may be any according to requirements.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. An apparatus for administering oxygen or air with added oxygen, for respiratory therapies, **characterized in that** it comprises a a Venturi meter (2) which is connected to an oxygen source (15), an air intake (16) which can be controlled by a flow control element (17) being provided on said Venturi meter (2), **characterized in that** said Venturi meter is directly connected to a respirator hood (1), and **in that** said Venturi meter (2) is connected to said oxygen source by means of a first flowmeter (14).

2. The apparatus according to claim 1, **characterized in that** said Venturi meter (2) is of the disposable type.

3. The apparatus according to the preceding claims, **characterized in that** it is of the portable type.

4. The apparatus according to the preceding claims, **characterized in that** said Venturi meter (2) is fixed to the delivery port (3) of said hood (1).

5. The apparatus according to one or more of the preceding claims, **characterized in that** said Venturi meter (2) has a body (10) which has, at its intake end, a coupling (11) to an intake (12) for connection to a first oxygen delivery hose (13), said intake openings (16) controlled by said flow control element (17) being provided at said coupling (11).

6. The apparatus according to claim 5, **characterized in that** said flow control element comprises a cap (17) which can be positioned on said coupling body (11) in order to control by blocking said openings (16).

7. The apparatus according to claim 5 or claim 6, **characterized in that** it comprises, on said body (10) of the Venturi meter (2), an auxiliary intake port (20) for the connection of a second oxygen delivery duct (21) which is controlled by a second flowmeter (22).

## Patentansprüche

1. Eine Vorrichtung zur Verabreichung von Sauerstoff oder damit angereicherter Luft für Atemwegstherapien, **dadurch gekennzeichnet, dass** sie einen Venturimesser (2) umfasst, der an eine Sauerstoffquelle (15) angeschlossen ist, einen Lufteinlass (16), der von einem an dem Venturimesser (2) bereitgestellten Durchflusssteuerungselement (17) gesteuert werden kann, **dadurch gekennzeichnet, dass** der Venturimesser direkt an eine Respirator-Haube (1) angeschlossen ist, und **dadurch**, dass der Venturimesser (2) über einen ersten Durchflussmesser (14) mit der Sauerstoffquelle verbunden ist.

2. Die Vorrichtung gemäß Anspruch 1, **dadurch gekennzeichnet, dass** der Venturimesser (2) vom Wegwerf-Typ ist.

3. Die Vorrichtung gemäß den obigen Ansprüchen, **dadurch gekennzeichnet, dass** sie vom tragbaren Typ ist.

4. Die Vorrichtung gemäß den obigen Ansprüchen, **dadurch gekennzeichnet, dass** der Venturimesser (2) an der Austrittsöffnung (3) der Haube (1) angebracht ist.

5. Die Vorrichtung gemäß einem oder mehreren der obigen Ansprüche, **dadurch gekennzeichnet, dass** der Venturimesser (2) einen Körper (10) hat, der an seinem Einlassende eine Kopplung (11) mit einem Einlass (12) zur Verbindung mit einem ersten Sauerstoff-Zufuhrschlauch (13) hat, wobei die Einlassöffnungen (16), die von dem Durchflusssteuerungselement (17) gesteuert werden, an der Kopplung (11) angebracht sind.

6. Die Vorrichtung gemäß Anspruch 5, **dadurch gekennzeichnet, dass** das Durchflusssteuerungselement eine Kappe (17) umfasst, die auf dem Kopplungskörper (11) positioniert werden kann, um die Öffnungen (16) durch Blockierung zu steuern.

7. Die Vorrichtung gemäß Anspruch 5 oder Anspruch 6, **dadurch gekennzeichnet, dass** sie, an dem Körper (10) des Venturimessers (2), eine zusätzliche Einlassöffnung (20) für den Anschluss eines zweiten Sauefstoff-Zufuhrkanals (21) umfasst, der von einem zweiten Durchflussmesser (22) gesteuert wird.

## Revendications

1. Appareil pour administrer de l'oxygène ou de l'air enrichi en oxygène, pour thérapies respiratoires, **caractérisé en ce qu'**il comprend un compteur Venturi (2) qui est raccordé à une source d'oxygène (15), une admission d'air (16) qui peut être contrôlée par un élément de régulation de débit (17) se trouvant sur ledit compteur Venturi (2), **caractérisé en ce que** ledit compteur Venturi est raccordé directement à un masque respiratoire (1) et **en ce que** ledit compteur Venturi (2) est raccordé à ladite source d'oxygène au moyen d'un premier débitmètre (14).

2. Appareil selon la revendication 1, **caractérisé en ce que** ledit compteur Venturi (2) est du type jetable.

3. Appareil selon les revendications précédentes, **caractérisé en ce qu'**il est du type portable.

4. Appareil selon les revendications précédentes, **caractérisé en ce que** ledit compteur Venturi (2) est fixé à l'orifice de sortie (3) dudit masque (1).

5. Appareil selon une ou plusieurs des revendications précédentes, **caractérisé en ce que** ledit compteur Venturi (2) possède un corps (10) qui présente, à son extrémité d'admission, un raccord (11) à une admission (12) pour raccordement à un premier conduit d'alimentation en oxygène (13), lesdites ouvertures d'admission (16) contrôlées par ledit élément de régulation de débit (17) étant prévues sut ledit raccord (11).

6. Appareil selon la revendication 5, **caractérisé en ce que** ledit élément de régulation de débit comprend un capuchon (17) qui peut être positionné sur ledit corps de raccord (11) afin de contrôler par blocage lesdites ouvertures (16).

7. Appareil selon la revendication 5 ou la revendication 6, **caractérisé en ce qu'**il comprend, sur ledit corps (10) du compteur Venturi (2), un orifice d'admission auxiliaire (20) pour le raccordement d'un second conduit d'alimentation en oxygène (21) qui est contrôlé par un second débitmètre (22).
